(19) Europäisches Patentamt

**European Patent Office**

**Office européen des brevets**

(11) Veröffentlichungsnummer: **0 1 1 5 063**
**B1**

(12) **EUROPÄISCHE PATENTSCHRIFT**

(45) Veröffentlichungstag der Patentschrift:
**27.01.88**

(21) Anmeldenummer: **83113050.5**

(22) Anmeldetag: **23.12.83**

(51) Int. Cl.⁴: **G 01 N 33/80,** G 01 N 33/577,
C 12 N 5/00, C 12 P 21/00

(54) Monoklonale Antikörper spezifisch für das humane Blutgruppenantigen k (cellano) und Hybridoma-Zell-Linien, die diese monoklonalen Antikörper produzieren.

(30) Priorität: **30.12.82 DE 3248618**

(43) Veröffentlichungstag der Anmeldung:
**08.08.84 Patentblatt 84/32**

(45) Bekanntmachung des Hinweises auf die Patenterteilung:
**27.01.88 Patentblatt 88/4**

(84) Benannte Vertragsstaaten:
**AT BE CH DE FR GB IT LI LU NL SE**

(56) Entgegenhaltungen:
**EP - A - 0 022 669**
**WO - A - 82/03089**
**GB - A - 2 113 713**

**CHEMICAL ABSTRACTS, Band 100, Nr. 23, 4. Juni 1984, Seiten 425-426, Nr. 189972r, Columbus, Ohio, US; D.J. ANSTEE et al.: "Monoclonal antibodies: tools for the characterization of blood group antigens"**
**JOURNAL OF IMMUNOLOGY, Band 129, Nr. 2, August 1982, Seiten 678-682, The American Association of Immunologists, Baltimore, Maryland, US; D.R. BUNDLE et al.: "Hybridomas specific for carbohydrates: synthetic human blood group antigens for the production, selection, and characterization of monoclonal typing reagents"**

(73) Patentinhaber: **Biotest-Serum-Institut GmbH,**
**Flughafenstrasse 4, D-6000 Frankfurt-Niederrad (DE)**

(72) Erfinder: **Sonneborn, H.H., Dr., Birkeneck 70,**
**D-6056 Heusenstamm (DE)**
Erfinder: **Pfeffer, Almut, Bornemann Strasse 20 I,**
**D-6000 Frankfurt 70 (DE)**
Erfinder: **Schulte, Gertrud, Odenwaldstrasse 32,**
**D-6000 Frankfurt-Nierrad (DE)**
Erfinder: **Mann, Gisela, Offenbacher Landstrasse 237,**
**D-6000 Frankfurt 70 (DE)**

(74) Vertreter: **Beil, Hans Christoph, Dr. et al, Beil, Wolff und Beil Rechtsanwälte Adelonstrasse 58, D-6230 Frankfurt am Main 80 (DE)**

**Beschreibung**

Die Erfindung betrifft monoklonale Antikörper spezifisch für das menschliche Blutgruppenantigen k (cellano), d.h. Antikörper mit Spezifität gegen das Blutgruppenmerkmal k humaner Erythrozyten sowie Hybridoma-Zell-Linien, die diese monoklonalen Antikörper produzieren.

Antikörper werden seit langem in der medizinischen Diagnostik verwendet. Ihre Verwendbarkeit ist jedoch etwas begrenzt, da es aufgrund ihrer Komplexizität und Vielseitigkeit schwierig ist, homogene Antikörper zu erzielen. Antikörper sind komplexe Proteinmoleküle bzw. Moleküle auf Proteinbasis, die durch das Immunsystem von Menschen oder Tieren produziert werden, um den Menschen oder das Tier gegen Antigene zu schützen. Antikörper für die medizinische Verwendung werden im allgemeinen dadurch erhalten, dass man einem Tier ein Antigen injiziert, welches das Immunsystem des Tieres stimuliert, und eine Antikörperfraktion aus dem peripheren Blutserum oder aus der Ascitesflüssigkeit isoliert.

Die Antikörperfraktion enthält Antikörper mit Spezifität gegen das injizierte Antigen sowie zahlreiche andere Antikörper, die von dem Tier produziert wurden. Nach bekannten Verfahren kann es möglich sein, im wesentlichen einen Antikörper zu isolieren mit Spezifität gegen das bestimmte Antigen. Jedoch selbst wenn ein Antikörper für ein bestimmtes Antigen isoliert wird, ist dieser Antikörper meistens ein Gemisch aus verschiedenen Antikörpern, die mit unterschiedlichen antigenen Determinanten des Antigens reagieren können.

Der Versuch, Zellen in vitro dazu zu bringen, in grösserer Menge und vor allem über längere Zeit hin spezifische Antikörper zu produzieren, scheiterte daran, dass die Zellen in vitro schnell inaktiviert werden und ihre Antikörperproduktion einstellen.

In den letzten Jahren wurden Verfahren zur Herstellung monoklonaler Antikörper entwickelt, die es möglich machen, homogene hochspezifische Antikörper zu erzielen. Im allgemeinen werden solche Antikörper dadurch hergestellt, dass man ein Tier mit einem Antigen immunisiert, Antikörper produzierende Zellen von diesem Tier erhält und die Antikörper produzierenden Zellen mit Tumor-Zell-Linien, z.B. Myeloma-Zell-Linien, fusioniert, wobei man Hybridomas erhält, die isoliert werden und die die monoklonalen Antikörper produzieren (Köhler, G. und Milstein, C., Eur. J. Immunol., 6, 511–519 (1976) und Köhler, G. und Milstein, C., Nature, 256, 495 (1975)).

Die Zellen des Hybridomas haben von dem einen «Elternteil» das Vermögen mitbekommen, einen ganz bestimmten Antikörper zu produzieren. Dem anderen «Elternteil», dem Myelom, verdanken sie ihre Fähigkeit, sich auf lange Zeit hin weiter zu teilen.

Diese Hybridomas können entweder in vitro oder aber als Tumoren in einem Wirtstier gezüchtet werden.

Da jede Antikörper produzierende Zelle (Klon) nur Antikörper einer einzigen Spezifität bildet, produzieren die monoklonalen Kulturen der Hybridomas, die aus einem solchen Klon mit einer Spezifität gezüchtet wurden, jeweils einen homogenen Antikörper, der entweder aus dem Kulturmedium von Hybridoma-Kulturen in vitro oder aus den Zellen, Ascitesflüssigkeiten oder Serum von tumortragenden Wirtstieren erhalten werden kann.

Nicht alle Hybridomaklone, die aus der Fusionierung von neoplastischen Zellen mit Antikörper produzierenden Zellen resultieren, sind spezifisch für das gewünschte Antigen, da viele Hybridomas Antikörper erzeugen, die das immunisierte Tier gegen andere Antigene produziert hat. Selbst Antikörper gegen spezifisches Antigen können von Klon zu Klon unterschiedlich sein, da Antikörper von unterschiedlichen Zellen mit unterschiedlichen antigenen Determinanten des gleichen Moleküls reagieren können. Deshalb ist es erforderlich, den entstehenden Antikörper oder das die Antikörper enthaltende Medium, Serum oder Ascitesflüssigkeit auf seine Reaktivität mit dem bestimmten Antigen zu testen und seine Spezifität zu testen, indem man bestimmt, mit welchen anderen Antigenen er reagiert.

Aufgrund der Homogenität und hohen Spezifität der auf diese Weise hergestellten monoklonalen Antikörper sind letztere äusserst wertvoll für diagnostische Zwecke.

Monoklonale Antikörper mit Spezifität gegen Blutgruppenmerkmale A und B sind bereits bekannt und lassen sich zur Bestimmung dieser Blutgruppenmerkmale innerhalb der Transfusionsmedizin und für Forensische Untersuchungen verwenden (vgl. VOAK, D., SACKS, S., ALDERSON, T., TAKEI, F., LENNOX, E., JARVIS, J., MILSTEIN, C. und DARNBOROUGH, J. (1980); Monoclonal Anti-A from a Hybrid Myeloma: Evaluation as a Blood Grouping Reagent. Vox Sanguinis, 39, 134–140).

Es bestand jedoch ein Bedarf, Antikörper mit Spezifität gegen andere Blutgruppenmerkmale humaner Erythrozyten herzustellen.

Erfindungsgemäss lassen sich nunmehr monoklonale Antikörper mit Spezifität gegen das Blutgruppenmerkmal k (cellano) von Humanerythrozyten herstellen. Bezüglich der Definition von k bzw. cellano siehe O. Prokop, W. Göhler «Die menschlichen Blutgruppen», Gustav-Fischer-Verlag, New York, 1976, Seiten 101–104.

Um die Produktion von Antikörpern mit Spezifität gegen das Blutgruppenmerkmal k zu induzieren, werden Tiere mit humanen k-positiven Erythrozyten immunisiert. Die dafür ausgewählte Tierspecies ist nicht kritisch, jedoch sollte der Stamm des Tieres genetisch gut definiert sein. Da dies für verschiedene Murinstämme, z.B. Ratten, Mäuse usw., zutrifft und da verschiedene neoplastische Zellen von Murinen ebenfalls als gut charakterisierte Kulturen erhältlich sind, wurden Mäuse für die Herstellung der erfindungsgemässen Antikörper verwendet. Jedoch lassen sich auch andere Tierspezies für diesen Zweck verwenden.

BALB/c Mäuse werden immunisiert mit humanen k-positiven Erythrozyten intravenös. Nach der letzten Injektion werden die Mäuse getötet und die Milzzellen für das Fusionsexperiment gewonnen.

Danach wird eine Milzzellensuspension hergestellt. Die Milzzellensuspension wird dann gewaschen, zweckmässigerweise durch Zentrifugieren in Hank's Medium. Die so erhaltenen Antikörper produzierenden Zellen werden mit neoplastischen Zellen fusioniert. Einige Tumorzellen, insbesondere Myeloma-Zellen, lassen sich vorteilhafterweise mit den Antikörper produzierenden Zellen fusionieren und liefern lebensfähige, Antikörper produzierende Kulturen von Hybridomas. Vorzugsweise sollten die Tumorzellen und die Antikörper produzierenden Zellen von der gleichen Species stammen, da dabei die Wahrscheinlichkeit, dass die genetischen und biochemischen Eigenschaften der Elternzellen verträglich sind, grösser wird und dadurch lebensfähige Hybridomas produziert werden.

Es gibt eine Vielzahl gut charakterisierter Myeloma-Zell-Linien, die sich erfindungsgemäss verwenden lassen. Beispiele hierfür sind P3-X63-Ag8, P3-X63-Ag8.653, S. 194, Y3, SP2/0, MPC-11 und deren Derivate. Vorzugsweise wird die Linie P3-X63-Ag8.653 (CNCM I-251) (Kearney et al., J. Immunol. 123: 1548 (1979)) verwendet. Vorzugsweise wählt man eine Myeloma-Linie aus, die keinen Antikörper produziert, so dass das dabei entstehende Hybridoma nur Antikörperketten von der Eltern-Milz- oder -Lymphknoten-Zelle produziert.

Die Myeloma-Zellen werden zweckmässigerweise in RPMI 1640 gehalten, dem 10% FCS zugesetzt werden. Myeloma-Zellen und Zellen, die von Mäusen erhalten wurden, die mit humanen k-positiven Erythrozyten immunisiert worden waren, werden nach der Methode von Köhler, G. und Milstein, C., Eur. J. Immunol. 6, 292 (1976) fusioniert.

Die Hybridomas werden durch die Verwendung von Hypoxanthin-Aminopterin-thymidin (HAT) Medium selektiert, wobei jegliches Wachstum in HAT-Medium die erfolgreiche Hybridisierung von Maus-Milz-Zellen und Maus-Myloma-Zellen anzeigt. Ihre Produktion von Antikörpern gegen die zur Immunisierung der Maus verwendeten k-positiven Erythrozyten wird mit Hilfe von Haemagglutinationstests festgestellt. Hybridzellen werden mit Hilfe der «limiting dilution method» in Mikrotiterplatten kloniert.

Klone von Hybridomas können in vitro gezüchtet werden nach den bekannten Gewebekulturmethoden, wie beispielsweise durch Cotton et al., Eur. J. Immunol. 3, 136 (1973) beschrieben. Wahlweise lassen sich die Hybridomas jedoch auch in vivo als Tumoren in einem histokompatiblen Tier oder in athymischen Nacktmäusen züchten. Die Antikörper lassen sich aus dem in vitro Kulturmedium oder aus dem Serum oder der Ascitesflüssigkeit des Tieres nach bekannten Methoden gewinnen.

Die Spezifität des Antikörpers von jedem Klon gegen das Blutgruppenmerkmal k in Humanerythrozyten wird mit Hilfe von Haemagglutinationstests festgestellt, und Klone, die Antikörper produzieren mit Spezifität gegen das Blutgruppenmerkmal k in Humanerythrozyten, werden selektiert.

Beispiel
Herstellung von Hybridoma-Zell-Linien

BALB/c Mäuse wurden immunisiert mit $5 \times 10^8$ humanen k-positiven Erythrozyten intravenös, $2 \times$ wöchentlich mit insgesamt 10 Injektionen. 4 Tage nach der letzten Injektion wurden die Mäuse getötet und die Milzzellen für das Fusionsexperiment gewonnen.

Danach wurde eine Milzzellensuspension hergestellt. Die Milzzellensuspension wurde $2 \times$ durch Zentrifugieren ($800 \times g$) in Hank's Medium gewaschen.

Hybridoma-Zell-Linien wurden erhalten durch Fusion von $10^8$ der auf vorstehende Weise gewonnenen Milzzellen mit $10^7$ Mausmyeloma-Zellen P3-X63-Ag8.653 (CNCM I-251), die in RPMI 1640 gehalten wurden, dem 10% FCS zugesetzt wurde.

Die Zellmischung wurde bei $800 \times g$ $2 \times$ zentrifugiert, resuspendiert zur Fusion in 40% (v/v)iger Polyethylenglykol (PEC) 4000-Lösung in Hank's Medium. Die Hybridomas wurden durch die Verwendung von HAT-Medium auf folgende Weise selektiert und auf Antikörper mit Spezifität gegen das Blutgruppenmerkmal k untersucht. Es wurden 570 Näpfe einer Mikrotiterplatte, gefüllt mit 200 µl Zellsuspension und einer Zelldichte von $1,3 \times 10^5$ Zellen/ml, ausgesät. Nach 12 Tagen Inkubation bei 37 °C und 5% $CO_2$ wurden in 224 Näpfen Klone gefunden.

Von den 224 Näpfen wurden von 174 Näpfen Überstände auf Antikörper gegen Blutgruppenantigene untersucht.

Die Überstände wurden im direkten Agglutinationstest mit dem bekannten Röhrchenzentrifugationstest und dem Anti-Human Globulin-Test untersucht.

Einer dieser Überstände zeigte bei Verwendung der Anti-Human Globulin-Tests eine Spezifität für das Blutgruppenantigen k.

Dieser Napf wurde nach der «limiting dilution» Methode kloniert. Insgesamt wurden 384 Mikrotiterplattennäpfe ausgesät. 316 davon zeigten Klonwachstum, in 70 davon wuchs nur 1 Klon. Diese 70 Näpfe wurden auf Spezifität getestet: 66 von 70 Näpfen waren positiv für das Merkmal k.

Die Tabelle zeigt eine typische Reaktion der erhaltenen Hybridomaüberstände mit verschiedenen Testzellen.

Hybridomas, die Antikörper mit dem in der Tabelle angegebenen Reaktionsmuster zeigten, wurden mit der «Limiting dilution method» kloniert.

So erhaltene Hybridoma-Linien wurden etabliert und sowohl in Überständen als auch in Ascitesflüssigkeiten, die nach Inocculation der Hybridoma-Linien in BALB/c-Mäusen gewonnen wurden, auf Spezifität und Avidität mit Test-Erythrozyten untersucht.

Die erhaltene Hybridoma-Linie ist unter der Nr. BS45 (CNCM I-268) etabliert und hinterlegt.

# Antikörper-Identifizierungstabelle

| Blutgruppensystem Donor No. | Rh-Typ | | D | C | E | c | e | Cw | I Zentrifug-Test | II 37° | III Coombs | Zusatz-tests | K | k | Kpa | Kpb | Fya | Fyb | Lua | Lub | Jka | Jkb | M | N | S | s | Lea | Leb | P1 | Xga | Coa | Cob | Doa | Dob | | Spezial Type |
|---|---|---|---|---|---|---|---|---|---|---|---|---|---|---|---|---|---|---|---|---|---|---|---|---|---|---|---|---|---|---|---|---|---|---|---|---|---|
| 107 675 | $R_1R_1$ | 1 | + | + | O | O | + | O | | | 4w | | O | + | + | + | O | + | O | + | O | + | + | + | O | + | + | O | +s | + | + | O | + | / | 1 | B7 Bg(a+) |
| 319 314 | $R_2R_2$ | 2 | + | O | + | + | O | O | | | 4w | | O | + | O | + | O | + | O | + | + | + | + | + | + | + | O | + | + | + | / | O | / | / | 2 | |
| 301 146 | $R^wR_1$ | 3 | + | + | O | O | + | + | | | 3 | | O | + | O | + | + | + | O | + | + | O | + | O | + | O | + | O | + | + | + | O | O | / | 3 | |
| 301 159 | $R_0r$ | 4 | + | O | O | + | + | O | | | 3 | | O | + | O | + | + | + | + | + | O | + | O | + | + | + | O | + | O | + | + | O | O | / | 4 | |
| 122 600 | r'r | 5 | O | + | O | + | + | O | | | — | | + | O | O | + | + | O | O | + | + | + | + | O | + | O | O | + | O | + | + | O | O | / | 5 | A28 Bg(c+) Rd+ |
| 121 943 | r''r | 6 | O | O | + | + | + | O | | | 3 | | O | + | O | + | + | O | O | + | + | + | + | O | O | + | O | + | + | O | + | O | + | / | 6 | |
| 120 325 | rr | 7 | O | O | O | + | + | O | | | 3w | | + | + | O | + | O | + | O | + | O | + | + | O | O | + | O | O | + | + | + | O | + | / | 7 | |
| 320 228 | rr | 8 | O | O | O | + | + | O | | | 4 | | O | + | O | + | + | + | O | + | O | + | + | O | + | + | O | + | +w | + | + | O | / | / | 8 | |

+ = positiv    O = negativ    / = nicht bestimmt    w = schwach    s = stark

Agglutinationsstärke
der positiven
Überstände (1 = schwach; 4 = stark; — = negativ)

0 115 063

Die Hybridoma-Linie BS45 (CNCM I-268) produziert einen Antikörper der Klasse IgG 1. Die Linie BS45 (CNCM I-268) produziert diesen Antikörper permanent in Zellsuspensionskulturen in vorzugsweise RPMI 1640-Medium.

Die Hybridoma-Linie BS45 (CNCM I-268) wächst auch als intraperitonealer Tumor in BALB/c-Mäusen und produziert unter diesen Bedingungen IgG 1 Antikörper mit der beschriebenen Spezifität, die aus dem Ascites der tumortragenden Tiere gewonnen werden können.

Die erfindungsgemässen Antikörper sind wertvolle Hilfsmittel für die Transfusionsmedizin und für Forensische Untersuchungen.

### Patentansprüche für die Vertragsstaaten: BE, CH, DE, FR, GB, IT, LI, LU, NL, SE

1. Hybridoma-Zell-Linie, die einen Antikörper mit Spezifität gegen ein Blutgruppenmerkmal von humanen Erythrozyten produziert, erhalten durch Fusion einer Antikörper produzierenden Tierzelle und einer neoplastischen Zelle, dadurch gekennzeichnet, dass sie einen Antikörper mit Spezifität gegen das Blutgruppenmerkmal k humaner Erythrozyten produziert.

2. Zell-Linie nach Anspruch 1, dadurch gekennzeichnet, dass die Antikörper produzierende Zelle murinen Ursprungs ist.

3. Zell-Linie nach einem der vorstehenden Ansprüche, dadurch gekennzeichnet, dass die Antikörper produzierenden Zellen BALB/c Maus-Milzzellen sind.

4. Zell-Linie nach einem der vorstehenden Ansprüche, dadurch gekennzeichnet, dass die neoplastische Zelle eine Myeloma-Zelle ist.

5. Zell-Linie nach einem der vorstehenden Ansprüche, dadurch gekennzeichnet, dass die neoplastische Zelle die Myeloma-Zell-Linie P3-X63-Ag8.653 (CNCM I-251) ist, die keine Antikörper produziert.

6. Hybridoma-Zell-Linie BS45 (CNCM I-268).

7. Monoklonaler Antikörper, gekennzeichnet durch seine Spezifität gegen das Blutgruppenmerkmal k humaner Erythrozyten.

8. Monoklonaler Antikörper nach Anspruch 7, dadurch gekennzeichnet, dass er von einer Hybridoma-Zell-Linie gemäss Anspruch 1 bis 6 produziert worden ist.

9. Verwendung des Antikörpers nach Anspruch 7 oder 8 zur Bestimmung des Blutgruppenmerkmals k humaner Erythrozyten.

### Patentansprüche für den Vertragsstaat AT

1. Verfahren zur Herstellung einer Hybridoma-Zell-Linie, die einen Antikörper mit Spezifität gegen das Blutgruppenmerkmal k humaner Erythrozyten produziert, dadurch gekennzeichnet, dass man eine Fusion einer Antikörper produzierenden Tierzelle und einer neoplastischen Zelle durchführt.

2. Verfahren nach Anspruch 1, dadurch gekennzeichnet, dass die Antikörper produzierende Zelle murinen Ursprungs ist.

3. Verfahren nach einem der vorstehenden Ansprüche, dadurch gekennzeichnet, dass die Antikörper produzierenden Zellen BALB/c Maus-Milzzellen sind.

4. Verfahren nach einem der vorstehenden Ansprüche, dadurch gekennzeichnet, dass die neoplastische Zelle eine Myeloma-Zelle ist.

5. Verfahren nach einem der vorstehenden Ansprüche, dadurch gekennzeichnet, dass die neoplastische Zelle die Myeloma-Zell-Linie P3-X63-Ag8.653 (CNCM I-251) ist, die keine Antikörper produziert.

6. Verfahren nach einem der vorstehenden Ansprüche, dadurch gekennzeichnet, dass man die Hybridoma-Zell-Linie BS45 (CNCM I-268) herstellt.

7. Verfahren zur Herstellung eines monoklonalen Antikörpers mit Spezifität gegen das Blutgruppenmerkmal k humaner Erythrozyten, dadurch gekennzeichnet, dass man eine gemäss einem der vorstehenden Ansprüche hergestellte Hybridoma-Zell-Linie inkubiert und den gewünschten Antikörper selektiert.

8. Verwendung des Antikörpers nach Anspruch 7 zur Bestimmung des Blutgruppenmerkmals k humaner Erythrozyten.

### Claims for the contracting states BE, CH, DE, FR, GB, IT, LI, LU, NL, SE

1. A hybridoma cell line which produces an antibody with specificity against a blood group feature of human erythrocytes, obtained by fusion of an antibody-producing animal cell and a neoplastic cell, characterized in that it produces an antibody with specificity against the blood group feature k of human erythrocytes.

2. A cell line as claimed in Claim 1, characterized in that the antibody-producing cell is of murine origin.

3. A cell line as claimed in any of the preceding Claims, characterized in that the antibody-producing cells are BALB/c mouse spleen cells.

4. A cell line as claimed in any of the preceding Claims, characterized in that the neoplastic cell is a myeloma cell.

5. A cell line as claimed in any of the preceding Claims, characterized in that the neoplastic cell is the myeloma cell line P3-X63-Ag8.653 (CNCM I-251) which does not produce antibodies.

6. Hybridoma cell line BS45 (CNCM I-268).

7. A monoclonal antibody, characterized by its specificity against the blood group feature k of human erythrocytes.

8. A monoclonal antibody as claimed in Claim 7, characterized in that it has been produced from a hybridoma cell line of the type claimed in Claims 1 to 6.

9. The use of the antibody claimed in Claim 7 or 8 for determining the blood group feature k of human erythrocytes.

### Claims for the contracting state AT

1. A process for the preparation of a hybridoma cell line which produces an antibody with specificity against the blood group feature k of human erythrocytes, characterized in that an antibody-producing animal cell and a neoplastic cell are fused.

2. A process as claimed in Claim 1, characterized in that the antibody-producing cell is of murine origin.

3. A process as claimed in any of the preceding Claims, characterized in that the antibody-producing cells are BALB/c mouse spleen cells.

4. A process as claimed in any of the preceding Claims, characterized in that the neoplastic cell is a myeloma cell.

5. A process as claimed in any of the preceding Claims, characterized in that the neoplastic cell is the myeloma cell line P3-X63-Ag8.653 (CNCM I-251) which does not produce antibodies.

6. A process as claimed in any of the preceding Claims, characterized in that the hybridoma cell line BS45 (CNCM I-268) is prepared.

7. A process for the preparation of a monoclonal antibody with specificity against the blood group feature k of human erythrocytes, characterized in that a hybridoma cell line prepared in accordance with any of the preceding Claims is incubated and the desired antibody selected.

8. The use of the antibody according to Claim 7 for determining the blood group feature k of human erythrocytes.

**Revendications pour les états contractants: BE, CH, DE, FR, GB, IT, LI, LU, NL, SE**

1. Lignée cellulaire d'hybridomes, qui produit un anticorps présentant une spécificité contre un facteur de groupe sanguin d'érythrocytes humains, obtenue par fusion d'une cellule animale produisant des anticorps et d'une cellule néoplasique, caractérisée en ce qu'elle produit un anticorps présentant une spécificité contre le facteur de groupe sanguin k d'érythrocytes humains.

2. Lignée cellulaire selon la revendication 1, caractérisée en ce que la cellule produisant des anticorps est d'origine murine.

3. Lignée cellulaire selon l'une des revendications précédentes, caractérisée en ce que les cellules produisant des anticorps sont des cellules de rate de souris Balb/c.

4. Lignée cellulaire selon l'une des revendications précédentes, caractérisée en ce que la cellule néoplasique est une cellule de myélome.

5. Lignée cellulaire selon l'une des revendications précédentes, caractérisée en ce que la cellule néoplasique est la lignée cellulaire de myélome P3-X63-Ag8.653 (CNCM I-251), qui ne produit pas d'anticorps.

6. Lignée cellulaire d'hybridomes BS45 (CNCM I-268).

7. Anticorps monoclonal, caractérisé par sa spécificité contre le facteur de groupe sanguin k d'érythrocytes humains.

8. Anticorps monoclonal selon la revendication 7, caractérisé en ce qu'il a été produit à partir d'une lignée cellulaire d'hybridome selon les revendications 1 à 6.

9. Utilisation de l'anticorps selon la revendication 7 ou 8 pour déterminer le facteur de groupe sanguin k d'érythrocytes humains.

**Revendications pour l'état contractant: AT**

1. Procédé pour la préparation d'une lignée cellulaire d'hybridomes, qui produit un anticorps présentant une spécificité contre le facteur de groupe sanguin k d'érythrocytes humains, caractérisé en ce qu'on réalise une fusion d'une cellule animale produisant des anticorps et d'une cellule néoplasique.

2. Procédé selon la revendication 1, caractérisé en ce que la cellule produisant des anticorps est d'origine murine.

3. Procédé selon l'une des revendications précédentes, caractérisé en ce que les cellules produisant des anticorps sont des cellules de rate de souris Balb/c.

4. Procédé selon l'une des revendications précédentes, caractérisé en ce que la cellule néoplasique est une cellule de myélome.

5. Procédé selon l'une des revendications précédentes, caractérisé en ce que la cellule néoplasique est la lignée cellulaire de myélome P3-X63-Ag8.653 (CNCM I-251), qui ne produit pas d'anticorps.

6. Procédé selon l'une des revendications précédentes, caractérisé en ce qu'on prépare la lignée cellulaire d'hybridomes BS45 (CNCM I-268).

7. Procédé pour la préparation d'un anticorps monoclonal présentant une spécificité contre le facteur de groupe sanguin k d'érythrocytes humains, caractérisé en ce qu'on incube une lignée cellulaire d'hybridomes préparée selon l'une des revendications précédentes, et qu'on sélectionne l'anticorps voulu.

8. Utilisation de l'anticorps selon la revendication 7 pour déterminer le facteur de groupe sanguin k d'érythrocytes humains.